**Europäisches Patentamt**

**European Patent Office**

**Office européen des brevets**

(19)

(11) Veröffentlichungsnummer: **0 062 026**
**B1**

(12) **EUROPÄISCHE PATENTSCHRIFT**

(45) Veröffentlichungstag der Patentschrift:
**06.11.85**

(21) Anmeldenummer: **82890040.7**

(22) Anmeldetag: **18.03.82**

(51) Int. Cl.⁴: **C 12 N 9/42, C 12 M 1/00**

(54) Verfahren zur Gewinnung von Cellulase und Anlage zur Durchführung des Verfahrens.

(30) Priorität: **25.03.81 AT 1393/81**

(43) Veröffentlichungstag der Anmeldung:
**06.10.82 Patentblatt 82/40**

(45) Bekanntmachung des Hinweises auf die Patenterteilung:
**06.11.85 Patentblatt 85/45**

(84) Benannte Vertragsstaaten:
**BE CH DE FR GB IT LI LU NL SE**

(56) Entgegenhaltungen:
**DE - A - 2 728 353**
**FR - A - 857 313**
**FR - A - 1 085 403**
**US - A - 3 616 222**
**US - A - 3 972 775**

(73) Patentinhaber: **STEYRERMÜHL Papierfabriks- und Verlags-Aktiengesellschaft, Fabriksplatz 1, A-4662 Steyrermühl (AT)**

(72) Erfinder: **Schwarzi, Karl, Dr., Vlilenstrasse 19, A-4662 Steyrermühl (AT)**
Erfinder: **Esterbauer, Hermann, Prof. Dr., Ziegelstrasse 21 w, A-8045 Graz (AT)**
Erfinder: **Schurz, Josef, Prof. Dr., Marlatroststrasse 172 f, A-8044 Graz (AT)**
Erfinder: **Jungschaffer, Gerald, Dipl.-Ing. Dr., Brunntalstrasse 13 b, A-4662 Steyrermühl (AT)**
Erfinder: **Meindl, Norbert, Dipl.-Ing. Dr., Lannastrasse 11, A-4810 Gmunden (AT)**

(74) Vertreter: **Wolfram, Gustav, Dipl.-Ing., Schwindgasse 7 P.O. Box 205, A-1041 Wien (AT)**

## Beschreibung

Die Erfindung betrifft ein Verfahren zur Gewinnung von Cellulase durch Züchten von Cellulase produzierenden Mikroorganismen auf cellulosehaltigem Kulturmedium in Gegenwart von Nährsalzen, wobei eine Suspension des zerkleinerten cellulosehaltigen Ausgangsmaterials in wässeriger Lösung mit den Mikroorganismen beimpft, die Kultur submers, vorzugsweise unter Belüftung, in Fermentern entwickelt, die Feststoffe aus der Kultur abgetrennt und die verbleibende cellulasehaltige Lösung gegebenenfalls konzentriert wird, sowie eine Anlage zur Durchführung dieses Verfahrens.

Es ist bereits ein Verfahren dieser Art bekannt, wobei die Entwicklung der Kultur in einer Mehrzahl von nebeneinander angeordneten und miteinander durch Leitungen verbundenen Fermentern erfolgt. Bei diesem sogenannten «Merry-go-round»-System wird der erste Fermenter zu 80% seines gesamten Volumens mit cellulosehaltiger Suspension und Inoculum beschickt und eine Fermentation etwa 25 Stunden lang durchgeführt. Dann werden 10% des fermentierten Produktes als Inoculum dem zweiten Fermenter zugeführt, welcher mit cellulosehaltiger Suspension aufgefüllt wird. Dann wird der zweite Fermenter 25 Stunden lang betrieben und danach werden wieder 10% des erhaltenen Fermentates dem dritten Fermenter zugeführt usw. Bei diesem System wird also in jedem der Fermenter nacheinander das Beimpfen, Entwickeln und die eigentliche Produktion durchgeführt.

Der Nachteil dieses Prozesses, bei welchem in jedem Fermenter die Wachstums-, Anpassungs- und Produktionsphase nacheinander verlaufen, besteht darin, dass eine zeit- und arbeitsaufwendige Vorbereitung jedes einzelnen Fermenters, nämlich Sterilisieren, Beimpfen, Anzüchten, durchgeführt werden muss, wodurch die Produktionsausbeute vermindert wird. Auch ein erheblicher Kostenaufwand ist mit einem solchen Produktionssystem verbunden.

Cellulase ist ein Enzym, welches zur Hydrolyse von cellulosehaltigem Ausgangsmaterial verwendet werden kann, wobei die Cellulose bis zu Monosacchariden abgebaut wird.

Es ist bekannt, dass verschiedene Mikroorganismen, wie aerobe und anaerobe Bakterien, Actinomycetes und Fungi Cellulase produzieren, wobei wichtige Vertreter der Gattung Trichoderma angehören. Insbesondere Trichoderma reesei-Stämme haben sich als geeignet erwiesen.

Die von den verschiedenen Mikroorganismen in das Substrat ausgeschiedenen Cellulasen unterscheiden sich voneinander in ihrer Zusammensetzung, u.zw. hinsichtlich C-1, C-x Enzymen und β-Glucosidase. Man spricht von sogenannten unvollständigen und vollständigen Cellulasen, d.h. solchen, welche Cellulose nur bis zu bestimmten Bruchstücken, und solchen, welche Cellulose bis zu Monosacchariden abbauen können.

Bisher wurde Cellulase auf mikrobiologischem Weg allerdings zumeist emers hergestellt, wobei die Züchtung der Mikroorganismen in Oberflächenkulturen, z.B. auf Weizenkleie, der fallweise Cellulosepulver zugesetzt wurde, unter Belüftung erfolgte.Ein solches Verfahren ist als Koji-Verfahren bekannt (Biotechnology and Bioengineering, Symposionsbericht 5.6 [1976], Verlag John Wiley·& Sons).

Nachteile bei dieser emersen Produktion bestehen darin, dass man nur diskontinuierlich arbeiten kann, dass teure Hilfsstoffe, wie Pepton, Eiweisshydrolysate oder Emulgatoren sowie Spurenelemente eingesetzt werden müssen, dass die Aufarbeitung durch Auslaugen der Oberflächenkulturen aufwendig und langwierig ist und dass der Platzbedarf für entsprechende Anlagen gross ist.

Aus der US-A-3 616 222 ist ein Verfahren zur Verzuckerung von Cellulose mittels Mikroorganismen (Fungi) oder mittels von letzteren erzeugten Enzymen bekannt. Der Fermentationsprozess wird unter Einsatz zweier verschiedener Klassen von Mikroorganismen durchgeführt, welche jeweils unvollständige Cellulasen produzieren. Der Fermentationsprozess kann unter bestimmten Umständen diskontinuierlich zweistufig durchgeführt werden, wobei in den beiden getrennten Stufen jeweils mit verschiedenen Mikroorganismen bzw. Enzymen gearbeitet wird. Auch bei diesem bekannten Verfahren müssen somit in jeder der beiden Stufen sämtliche Wachstums- und Produktionsphasen hintereinander ablaufen, ohne dass einzelne Phasen spezifisch begünstigt werden könnten.

Die Erfindung bezweckt die Vermeidung der beschriebenen Nachteile bekannter Verfahren und stellt sich die Aufgabe, ein Verfahren zu schaffen, welches kontinuierlich durchgeführt werden kann, wobei in jedem Fermenter eine bestimmte Wachstums- bzw. Produktionsphase unter Konstanthalten optimaler Bedingungen einfach und sicher eingestellt werden können und wobei auch die Belüftung unter Vermeiden von Infektionen des Substrates durch Fremdorganismen leichter durchführbar ist.

Gemäss der Erfindung wird diese Aufgabe bei einem Verfahren der eingangs definierten Art dadurch gelöst, dass die Entwicklung der beimpften Kultur in vier aufeinanderfolgenden Fermentationsstufen kontinuierlich mit von Stufe zu Stufe zunehmender Cellulase- und abnehmender Cellulosekonzentration durchgeführt wird, wobei in der ersten Fermentationsstufe ein pH-Wert von 5,5 bis 6 eingehalten wird, in der zweiten und dritten Fermentationsstufe der pH-Wert auf etwa 3,5 abgesenkt und in der letzten Fermentationsstufe der pH-Wert wieder auf etwa 4,8 erhöht wird, und dass unter Einhaltung einer konstanten Füllstandshöhe in allen Fermentern die gleiche Verweilzeit der Suspension eingestellt wird.

Die erste Fermentationsstufe erfolgt bei einem höheren pH-Bereich als die späteren. In dieser Phase findet vor allem ein optimales Wachstum des eingesetzten Mikroorganismus statt. In der zweiten und dritten Fermentationsstufe wird durch Herabsetzung des pH-Wertes eine optimale Produktion der C-1 und C-x Enzyme bewirkt. In der letzten Stufe wird der pH-Wert wieder erhöht, wodurch gewährleistet ist, dass β-Glucosidase produziert wird. Damit wird in der zweiten, dritten und vierten Phase bzw. Stufe eine vollständige Cellulase erzeugt, die Cellulose bis zu Monosacchariden abbauen kann.

Für jede der Fermentationsstufen können jeweils ein einziger oder mehrere Fermenter vorgesehen sein.

Das cellulosehaltige Ausgangsmaterial dient als Kohlenstoffquelle für die Mikroorganismen. Die Nährlösung wird aus handelsüblichen Mineraldüngern bereitet. Als Stickstoffquelle kommen für das erfindungsgemässe Verfahren neben Harnstoff und Harnstoffderivaten beispielsweise Ammonium enthaltende Stickstoffdünger, wie Ammoniumsulfat, -chlorid und -phosphat, in Frage. Als Phosphorquelle sind Phosphatdüngemittel, wie Superphosphat, Doppelsuperphosphat, Triple-Phosphat und Hyperphosphat geeignet. Auf die Zugabe von üblichen Spurenelementen kann verzichtet werden, da diese in dem zur Herstellung der Nährlösung verwendeten gewöhnlichen Leitungswasser sowie in den zugesetzten Düngemitteln als Begleitsubstanzen enthalten sind.

Da die meisten Cellulase produzierenden Mikroorganismen aerobe Bedingungen benötigen, wird das Kulturmedium vorzugsweise belüftet.

Das cellulosehaltige Ausgangsmaterial kann mit Nährsalzen und Frischwasser, vorteilhaft ozonisiertem Frischwasser, versetzt und die Suspension mechanisch aufgeschlagen sowie sterilisiert werden, bevor sie beimpft wird.

Zur Sterilisation kann Dampf, Ozon oder ein Biocid wie Pentachlorphenol-Natrium eingesetzt werden.

In jedem einzelnen Fermenter können die für die jeweilige Entwicklungsphase der Kultur optimalen Voraussetzungen hinsichtlich pH-Wert, Temperatur, Sauerstoffgehalt, Stoffdichte und Nährmedium eingestellt werden, wobei der pH-Wert insbesondere mit Ammoniak eingestellt wird. Ammoniak kann von den Mikroorganismen gleichzeitig auch als Stickstoffträger verwertet werden.

Nach einer vorteilhaften Ausführungsform des erfindungsgemässen Verfahrens wird die Suspension in Vorfermentern mit einem einzigen Mikroorganismusstamm aus der Gattung Trichoderma beimpft, wobei das in der Vorfermentation erhaltene Inoculum der ersten Fermentationsstufe lediglich zu Beginn des kontinuierlichen Produktionsprozesses zugeführt wird.

Auch die Vorfermentation zum Anzüchten der Mikroorganismen kann mehrstufig durchgeführt werden, wobei die Volumsverhältnisse der Vorfermenter in den einzelnen Stufen jeweils etwa 1:10 betragen können. Wird die Vorfermentation beispielsweise dreistufig durchgeführt, verhalten sich die Volumina der Vorfermenter und des ersten Hauptfermenters zueinander vorzugsweise wie 1:20:200:5000.

Eine Vorfermentation erwies sich zur Anreicherung der Zellen des Mikroorganismus als besonders günstig. Die Hauptfermenter können etwa gleichen Rauminhalt aufweisen.

Bei mesophilen Mikroorganismen wird zweckmässig in der Vorfermentation und in den Hauptfermentationsstufen eine Temperatur von 25 bis 50 °C eingehalten.

Die Ausgangs-Konzentration an cellulosehaltigem Material in der Suspension wird vorzugsweise bei 1 bis 10% Masse gehalten.

Nach einer weiteren bevorzugten Ausführungsform des erfindungsgemässen Verfahrens werden nach Abtrennung der Feststoffe aus der cellulasehaltigen Lösung der letzten Fermentationsstufe die Feststoffe hydrolysiert und das Hydrolysat als Nährsalzlösung der Ausgangsmischung zugefügt. So wird einer Überalterung des Mycels ständig entgegengearbeitet, u.zw. dadurch, dass aus dem ersten Fermenter ständig neues Mycel in die nächsten Fermenter bzw. Stufen geführt wird. Durch Rückführung des Hydrolysates werden wichtige Wuchsstoffe wieder in den Prozess eingebracht.

Vorteilhaft wird die cellulosehaltige Lösung aus der letzten Fermentationsstufe hinsichtlich ihres Cellulasegehaltes durch Ultrafiltration aufkonzentriert und die Cellulase aus dem Konzentrat mittels polarer Lösungsmittel ausgefällt. Als polares Lösungsmittel ist beispielsweise Aceton geeignet. Auf diese Weise werden Cellulase-Präparate in trockener Form erhalten.

Nach einer besonders vorteilhaften Ausführungsform wird aus den Fermentationsstufen abgezogenes Kulturfiltrat hinsichtlich seines Cellulasegehaltes durch Ultrafiltration aufkonzentriert und das Permeat zu den Fermentern rückgeführt. Mit steigender Cellulasekonzentration erniedrigt sich nämlich die Bildungsgeschwindigkeit von Cellulase, so dass eine laufende Entfernung von Cellulase günstig für den Prozessablauf ist. Das rückgeführte Permeat enthält noch Nährstoffe für die Mikroorganismen.

Eine erfindungsgemässe Anlage ist dadurch gekennzeichnet, dass hintereinander angeordnet sind: ein mit Rührwerk ausgestatteter Aufschlagbehälter zur Herstellung einer Cellulose und Nährsalze enthaltenden wässerigen Suspension, mindestens ein mit Rührwerken ausgestatteter Sterilisierbehälter, welchem ein Mischer mit einer Zuführung für Sterilisationsmittel vorgeschaltet ist, eine Vorfermentationseinrichtung, welche vorteilhaft eine Mehrzahl von Vorfermentern aufweist, die eine um jeweils etwa das 10-fache gegenüber dem vorangehenden Fermenter vergrösserte Aufnahmefähigkeit besitzen, und eine Hauptfermentationseinrichtung, die eine Mehrzahl von Fermentern aufweist, wobei die Vor- und Hauptfermenter Zuführungseinrichtungen für sauerstoffhaltige Gase, Rühr- bzw. Mischorgane, Entlüftungseinrichtungen, Regeleinrichtungen für Temperatur und pH-Wert und gegebenenfalls Schaumbrecher aufweisen, sowie weiters Einrichtungen zum Abtrennen der Feststoffe von der cellulasehaltigen Lösung, welche vorteilhaft Klärbehälter und Zentrifuge umfassen, gegebenenfalls eine Ultrafiltrationsanlage zum Konzentrieren der cellulasehaltigen Lösung sowie ein Behälter zur Fällung der Cellulase.

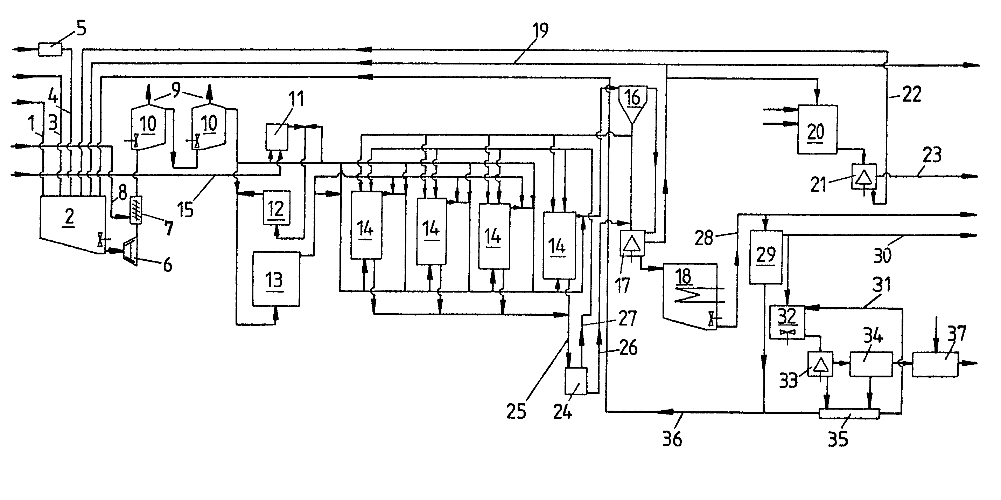

Die Erfindung wird im folgenden anhand des in der Zeichnung dargestellten – einer Ausführungsform der erfindungsgemässen Anlage entsprechenden – Verfahrensschemas und des Beispieles näher erläutert.

Zellstoff bzw. cellulosehaltiges Material wird zunächst trocken vorgemahlen und sodann durch die Zuführung 1 in einen Aufschlagbehälter 2 eingebracht. Nährsalze werden über die Zuführung 3 zugesetzt. Frischwasser gelangt durch die Leitung 4, in welche ein Ozonisator 5 zum Entkeimen des Wassers eingebaut ist, in den Aufschlagbehälter, worin mittels eines Rührwerkes eine Suspension hergestellt wird. Aus dem Aufschlagbehälter wird die Suspension einer Mühle 6 zugeführt, in welcher Defibrierung und Fibrillierung des Materials stattfindet. Anschliessend gelangt die Suspension in einen Mischer 7, in welchem sie mit einem Sterilisationsmittel aus der Zuführung 8 vermengt wird. Dem Mischer sind mit Entlüftungseinrichtungen 9 und Rührwerk ausgestattete Sterilisierbehälter 10 nachgeschaltet, in denen die Suspension während einer vorgegebenen Verweilzeit entkeimt wird.

Die sterilisierte Suspension wird sodann zu Vorfermentern bzw. zu Hauptfermentern gepumpt. Die Fermentationseinheit besteht in der dargestellten Ausführungsform aus drei Vorfermentern 11, 12, 13 und vier jeweils mit 14 bezeichneten, hintereinander geschalteten Hauptfermentern. Alle Fermenter sind mit Rühr- bzw. Mischorganen ausgerüstet, weiters mit Regeleinheiten für Temperatur und pH-Wert sowie mit Systemen zur Schaumbekämpfung. Weiters sind jeweils Belüftungen mit Sterilluft vorgesehen. Die aus den Fermentern tretende Abluft wird ebenfalls gefiltert.

Die Hauptfermenter werden mit konstanter Füllstandshöhe gefahren. Dem Vorfermenter 11 wird über die Zuführung 15 das Inoculum zugesetzt. Alle Fermenter sind über sterilisierbare Leitungen miteinander verbunden, wobei die Verbindungen zwischen den Hauptfermentern so angeordnet sind, dass sowohl ein diskontinuierlicher als auch ein kontinuierlicher Betrieb möglich ist. Sollte es aufgrund einer verminderten Wachstumsrate des Mikroorganismus notwendig sein, so können weitere Hauptfermenter in prinzipiell gleicher Weise zugeschaltet werden.

Bei kontinuierlichem Betrieb wird vom letzten Fermenter dessen gesamter Inhalt, nämlich Biomasse und Enzymlösung, abgezogen und in einen Klärbehälter 16 übergeführt. Hier erfolgt eine Vortrennung von enzymhaltiger Lösung und Biomasse, beispielsweise Pilzmycel. Die Biomasse kann zur Gänze oder teilweise zur Erhöhung der Zellkonzentration in die Hauptfermenter 14 zurückgeführt werden. Ein anderer Teil der Biomasse sowie die vorgeklärte Enzymlösung werden in einer Trenneinrichtung, beispielsweise einer Zentrifuge 17, in Festanteile und Kulturfiltrat aufgetrennt, und das Filtrat wird in einen mit Rühr- und Kühleinrichtung ausgestatteten Lagerbehälter 18 geleitet. Die abgetrennte Biomasse kann anschliessend aus der Anlage zu beliebiger Weiterverwendung abgezogen werden, sie kann aber auch in unbehandelter Form durch die Zuführung 19 zum Aufschlagbehälter 2 als Nährsalzträger rückgeführt werden. Die anfallende Biomasse ist eiweisshaltig und kann daher als Futtermittelzusatz, als Substratzusatz für andere mikrobiologische Prozesse aber auch als stickstoffhaltiges Düngemittel verwendet werden. Es ist jedoch vorzuziehen, die Biomasse in einem Autoklaven 20 mit Zuführungen für Dampf und Säure einer Vorhydrolyse zu unterwerfen. Die erhaltene Hydrolysatlösung wird – beispielsweise mittels Zentrifuge 21 – vom verbleibenden Feststoff abgetrennt und als Nährsalzlösung über die Leitung 22 rückgeführt. Durch Rückführung der Biomasse in unveränderter plasmolysierter Form oder Rückführung des Hydrolysates ist es nicht notwendig, zusätzliche Hilfsstoffe, wie Pepton, Eiweisshydrolysate oder Emulgatoren, wie etwa Polyoxyäthylenderivate von Sorbitanhydriden, die teilweise mit Fettsäure verestert sind, zu verwenden. Der über den Austrag 23 aus der Anlage entfernte Restfeststoff kann zur Gewinnung von Wärmeenergie verbrannt werden.

Im gezeigten Ausführungsbeispiel der erfindungsgemässen Anlage ist weiters eine Ultrafiltrationsanlage 24 vorgesehen, mittels welcher das gegebenenfalls aus den einzelnen Fermentern 14 über die Sammelleitung 25 abgezogene Kulturfiltrat hinsichtlich seines Cellulasegehaltes aufkonzentriert werden kann. Das Enzymkonzentrat wird über die Leitung 26 mit dem Kulturfiltrat aus dem Klärbehälter 16 vereinigt und der Zentrifuge 17 zugeführt, da sich im Konzentrat noch lebende Zellen befinden können, welche vor dem Lagerbehälter 18 abgetrennt werden müssen. Das Permeat wird durch die Leitung 27 wieder den Fermentern 14 zugesetzt. Durch diese Vorgangsweise erhöht sich die Zellkonzentration in den Fermentern 14, und gleichzeitig wird die Produktion von Cellulase beschleunigt.

Die Enzymlösung im Lagerbehälter 18 wird über die Leitung 28 abgezogen und kann entweder direkt weiter verwendet oder einer Ultrafiltrationsanlage 29 zugeführt werden. Das Konzentrat aus (29) kann als solches über die Leitung 30 seinem Einsatzort zugeführt werden oder in einem mit Rührer und Zuleitung 31 für ein Fällungsmittel ausgerüsteten Behälter 32 mit Fällungsmittel versetzt werden. Die Konzentrierung der Enzymlösung kann alternativ auch z.B. mittels Dünnschichtverdampfung vorgenommen werden. Das ausgefällte Enzym wird, nachdem es mittels einer Zentrifuge 33 oder eines Filters abgetrennt wurde, in eine Trocknungsanlage 34 übergeführt.

Die Lösung aus der Zentrifuge 33 wird gemeinsam mit den kondensierten Dämpfen aus der Trocknungsanlage 34 in einer Destillationsanlage 35 aufgearbeitet. Das wiedergewonnene Fällungsmittel wird über die Zuleitung 31 abgezogen, der wässerige Rückstand wird zusammen mit dem Permeat aus der Ultrafiltrationsanlage 29 über die Leitung 36 wieder dem Aufschlagbehälter 2 zugeführt, wodurch der Bedarf an Frischwasser erheblich eingeschränkt wird. Die Trocknungsanlage 34 kann beispielsweise als Sprühverdampfungs- oder Dünnschichtverdampfungsanlage ausgeführt sein. Im Anschluss an die Trocknung erfolgt die Einstellung der Aktivität des hergestellten Enzymtrockenpräparates durch Zugabe von Inertmaterial oder Milchzucker in einem Mischer 37. Erfindungsgemäss hergestellte Trockenpräparate weisen volle Cellulaseaktivität auf.

Beispiel

In den Aufschlagbehälter der voranstehend beschriebenen Anlage werden 30 kg Zellstoff, 1,5 kg Mycel von Trichoderma reesei aus der laufenden Produktion, 0,7 kg $KH_2PO_4$ und 1,5 kg $(NH_4)_2SO_4$ eingebracht und die Mischung durch Zusatz von ozonisiertem Wasser auf ein Volumen von 1000 l eingestellt. Nach Defibrieren, Fibrillieren und Sterilisation der Suspension während 20 Minuten bei 120 °C wird dieser Ansatz im ersten Hauptfermenter mit frischer Pilzmasse von Trichoderma reesei aus dem Vorfermenter 13 versetzt.

Die Verweilzeit in den hintereinander geschalteten Hauptfermentern beträgt jeweils 36 Stunden, die pH-Werte betragen:

| | |
|---|---|
| erster Hauptfermenter: | 5,5–6 |
| zweiter Hauptfermenter: | 3,5 |
| dritter Hauptfermenter: | 3,5 |
| vierter Hauptfermenter: | 4,8 |

der Temperaturverlauf ist folgender:

| | |
|---|---|
| erster Hauptfermenter: | 30 °C |
| zweiter Hauptfermenter: | 29 °C |
| dritter und vierter Hauptfermenter: | 28 °C |

zugeführte Luftmengen:

| | |
|---|---|
| erster Hauptfermenter: | 0,2 l/l Substanz·min |
| zweiter Hauptfermenter: | 0,25 l/l Substanz·min |
| dritter und vierter Hauptfermenter: | 0,35 l/l Substanz·min |

Der Feststoffgehalt in den Fermentern sinkt von 3% auf 2,9%, 1,8% und schliesslich 1%.

Im ersten Hauptfermenter liegt als Feststoff Cellulose vor, im vierten Hauptfermenter ist nur mehr Pilzmycel vorhanden, da die Cellulose durch das Zellwachstum verbraucht wird.

Im letzten (vierten) Hauptfermenter resultieren dann 1.000 l Lösung mit 2,8 FPU (Filterpapiereinheiten (früher FPD-activity-units; nach N. Toyama, Advances in Enzyme Hydrolysis of Cellulose and Related Materials, E.T. Reese, Ed. Pergamon Press, London 1963, Seite 235)) pro ml entsprechend 5,6 ml Protein/ml. Die Gesamtlösung enthält somit $2,8 \times 10^6$ FPU (5,6 kg Protein) und 9,6 kg Trichoderma-Mycelüberschuss.

Zum Aufkonzentrieren der Enzymlösung wurde die Ultrafiltration herangezogen. Verwendet wurde eine Kunststoffmembrane mit einer Ausschlussgrenze von 10.000. Es resultierte dabei ein Konzentrat mit einem Volumen von 50 l und einem Gehalt von $56 \times 10^6$ FPU. Dieses Konzentrat wurde bei Raumtemperatur mit 100 l Aceton versetzt und anschliessend das ausgefällte Enzym abzentrifugiert und im Luftstrom getrocknet. Das Resultat waren 5 kg Protein in Form eines weissen Pulvers, das $2,5 \times 10^6$ FPU Cellulase enthielt.

**Patentansprüche**

1. Verfahren zur Gewinnung von Cellulase durch Züchten von Cellulase produzierenden Mikroorganismen auf cellulosehaltigem Kulturmedium in Gegenwart von Nährsalzen, wobei eine Suspension des zerkleinerten cellulosehaltigen Ausgangsmaterials in wässeriger Lösung mit den Mikroorganismen beimpft, die Kultur submers, vorzugsweise unter Belüftung, in Fermentern entwickelt, die Feststoffe aus der Kultur abgetrennt und die verbleibende cellulasehaltige Lösung gegebenenfalls konzentriert wird, dadurch gekennzeichnet, dass die Entwicklung der beimpften Kultur in vier aufeinanderfolgenden Fermentationsstufen kontinuierlich mit von Stufe zu Stufe zunehmender Cellulase- und abnehmender Cellulosekonzentration durchgeführt wird, wobei in der ersten Fermentationsstufe ein pH-Wert von 5,5 bis 6 eingehalten wird, in der zweiten und dritten Fermentationsstufe der pH-Wert auf etwa 3,5 abgesenkt und in der letzten Fermentationsstufe der pH-Wert wieder auf etwa 4,8 erhöht wird, und dass unter Einhaltung einer konstanten Füllstandshöhe in allen Fermentern die gleiche Verweilzeit der Suspension eingestellt wird.

2. Verfahren nach Anspruch 1, dadurch gekennzeichnet, dass der pH-Wert mit Ammoniak eingestellt wird.

3. Verfahren nach Anspruch 1 oder 2, dadurch gekennzeichnet, dass die Suspension in Vorfermentern mit einem einzigen Mikroorganismusstamm aus der Gattung Trichoderma beimpft wird, wobei das in der Vorfermentation erhaltene Inoculum der ersten Fermentationsstufe lediglich zu Beginn des kontinuierlichen Produktionsprozesses zugeführt wird.

4. Verfahren nach den Ansprüchen 1 bis 3, dadurch gekennzeichnet, dass die Ausgangs-Konzentration an cellulosehaltigem Material in der Suspension bei 1 bis 10% Masse gehalten wird.

5. Verfahren nach den Ansprüchen 1 bis 4, dadurch gekennzeichnet, dass nach Abtrennung der Feststoffe aus der cellulasehaltigen Lösung der letzten Fermentationsstufe die Feststoffe hydrolysiert werden und das Hydrolysat als Nährsalzlösung der Ausgangsmischung zugefügt wird.

6. Verfahren nach Anspruch 5, dadurch gekennzeichnet, dass die cellulasehaltige Lösung durch Ultrafiltration hinsichtlich ihres Cellulasegehaltes aufkonzentriert und die Cellulase aus dem Konzentrat mittels polarer Lösungsmittel ausgefällt wird.

7. Verfahren nach den Ansprüchen 1 bis 6, dadurch gekennzeichnet, dass aus den Fermentationsstufen abgezogenes Kulturfiltrat hinsichtlich seines Cellulasegehaltes durch Ultrafiltration aufkonzentriert und das Permeat zu den Fermentern rückgeführt wird.

8. Anlage zur Durchführung des Verfahrens nach den Ansprüchen 1 bis 7, dadurch gekennzeichnet, dass hintereinander angeordnet sind: ein mit Rührwerk ausgestatteter Aufschlagbehälter (2) zur Herstellung einer Cellulose und Nährsalze enthaltenden wässerigen Suspension, mindestens ein mit Rührwerken ausgestatteter Sterilisierbehälter (10), welchem ein Mischer (7) mit einer Zuführung (8) für Sterilisationsmittel vorgeschaltet ist, eine Vorfermentationseinrichtung, welche vorteilhaft eine Mehrzahl von Vorfermentern (11, 12, 13) aufweist, die eine um jeweils etwa das 10-fache gegenüber dem vorangehenden Fermenter vergrösserte Aufnahmefähigkeit besitzen, und eine Hauptfermentationseinrichtung, die eine Mehrzahl von Fermentern (14) aufweist, wobei die Vor- und Hauptfermenter Zuführungseinrichtungen für sauerstoffhaltige Gase, Rühr- bzw. Mischorgane, Entlüftungseinrichtungen, Regeleinrichtungen für Temperatur und pH-Wert und gegebenenfalls Schaumbrecher aufweisen, sowie weiters Einrichtungen zum Abtrennen der Feststoffe von der cellulasehaltigen Lösung, welche vorteilhaft Klärbehälter (16) und Zentrifuge (17) umfassen, gegebenenfalls eine Ultrafiltrationsanlage (29) zum Konzentrieren der cellulasehaltigen Lösung sowie ein Behälter (32) zur Fällung der Cellulase.

**Revendications**

1. Procédé pour l'obtention de cellulase par culture de microorganismes producteurs de cellulase sur un milieu de culture contenant de la cellulose en présence de sels nutritifs, selon lequel une suspension de la matière de départ fractionnée contenant de la cellulose est inoculée en solution aqueuse avec les microorganismes, la culture est développée à l'état submergé, de préférence avec aération, dans des fermenteurs, les matières solides sont séparées de la culture et la solution résiduelle contenant de la cellulase est éventuellement concentrée, caractérisé en ce que l'on produit le développement de la culture inoculée en continu dans quatre stades successifs de fermentation, avec augmentation de la concentration de la cellulase et diminution de la concentration de cellulose d'un stade à l'autre et avec maintien du pH das le premier stade de fermentation à 5,5–6, abaissement du pH dans les second et troisième stades de fermentation à environ 3,5 et rétablissement du pH à 4,8 dans le dernier stade de fermentation, et que, en maintenant un niveau de remplissage constant dans tous les fermenteurs, on produit la même durée de séjour de la suspension dans tous les fermenteurs.

2. Procédé selon la revendication 1, caractérisé en ce que l'on ajuste le pH avec l'ammoniaque.

3. Procédé selon la revendication 1 ou 2, caractérisé en ce que l'on inocule la suspension dans des préfermenteurs avec une seule souche de microorganismes de l'espèce Trichoderma, l'inoculant obtenu dans la fermentation préalable étant seulement introduit dans le premier stade de fermentation au début du processus de production continu.

4. Procédé selon les revendications 1 à 3, caractérisé en ce que l'on maintient la concentration initiale en matière contenant de la cellulose dans la suspension entre 1 à 10% en poids.

5. Procédé selon les revendications 1 à 4, caractérisé en ce que, après séparation des matières solides de la solution contenant de la cellulose provenant du dernier stade de fermentation, on hydrolyse les matières solides et on ajoute l'hydrolysat comme solution saline nutritive au mélange de départ.

6. Procédé selon la revendication 5, caractérisé en ce que l'on concentre la solution contenant de la cellulase par ultrafiltration en ce qui concerne sa teneur en cellulase et on produit la précipitation de la cellulase du produit concentré au moyen d'un solvant polaire.

7. Procédé selon les revendications 1 à 6, caractérisé en ce que l'on concentre le filtrat de culture soutiré des stades de fermentation par ultrafiltration en ce qui concerne sa teneur en cellulase et on réintroduit le perméat dans les fermenteurs.

8. Installation pour la mise en œuvre du procédé selon les revendications 1 à 7, caractérisée en ce qu'elle comprend, disposés les uns à la suite des autres, un récipient de raffinage (2) équipé d'un dispositif d'agitation pour la production d'une suspension aqueuse contenant de la cellulose et des sels nutritifs au moins un récipient de stérilisation (10) équipé de dispositifs d'agitation et précédé d'un mélangeur (7) avec une alimentation (8) pour un agent de stérilisation, un dispositif de fermentation prélable, qui présente avantageusement plusieurs préfermenteurs (11, 12, 13) ayant chacun une capacité environ dix fois plus grande que le fermenteur précédent, et un dispositif de fermentation principale qui présente plusieurs fermenteurs (14), les préfermenteurs et les fermenteurs principaux étant pourvus de dispositifs d'alimentation avec des gaz contenant de l'oxygène, d'organes d'agitation et de mélange, de dispositifs d'échappement d'air, de dispositifs de réglage de la température du pH et éventuellement de dispositifs antimousse, de même que des dispositifs pour séparer les matières solides de la solution contenant de la cellulase, comprenant avantageusement des récipients de clarification (16) et des centrifugeuses (10) et éventuellement une installation d'ultrafiltration (29) pour concentrer la solution contenant de la cellulase, ainsi qu'un récipient (32) pour la précipitation de la cellulase.

**Claims**

1. A method for obtaining cellulase by growing cellulase-producing microorganisms on cellulose-containing culture medium in the presence of nutrient salts, wherein a suspension of the disintegrated cellulose-containing starting material in an aqueous solution is inoculated with the microorganisms, the culture is developed in a submersed state in fermenters, preferably under aeration, the solids are separated from the culture and the remaining cellulase-containing solution is concentrated, if desired, characterised in that the development of the inoculated culture is carried out in four consecutive fermentation stages continuously with the cellulase concentration increasing and the cellulose concentration decreasing from stage to stage, with a pH of 5.5 to 6 being maintained in te first fermentation stage, which pH is lowered to about 3.5 in the second and third fermentation stages and is again raised to about 4.8 in the last fermentation stage, and that, by observing a constant filling level in all of the fementers, the same dwell time of the suspension in the fermenters is adjusted.

2. A method according to claim 1, characterised in that the pH is ajusted with ammonia.

3. A method according to claim 1 or 2, characterized in that the suspension is inoculated with a single microorganism strain of the species trichoderma in pre-fermenters, the inoculum obtained in the pre-fermentation being supplied to the first fermentation stage merely at the beginning of the continuous production process.

4. A method according to claims 1 to 3, characterised in that the starting concentration of cellulose-containing material in the suspension is maintained at 1 to 10% by mass.

5. A method according to claims 1 to 4, characterised in that, after separation of the solids from the cellulase-containing solution of the last fermentation stage, the solids are hydrolyzed and the hydrolysate is added to the starting mixture as the nutrient salt solution.

6. A method according to claim 5, characterised in that the cellulase-containing solution is upgraded by ultrafiltration with respect to its cellulase content, the cellulase being precipitated from the concentrate by means of polar solvents.

7. A method according to claims 1 to 6, characterised in that culture filtrate withdrawn from the fermentation stages is upgraded by ultrafiltration with respect to its cellulase content, the permeate being recycled to the fermenters.

8. A plant for carrying out the method according to claims 1 to 7, characterised in that there are arranged in order: a pulping vessel (2) equipped with an agitating means for the production of an aqueous suspension containing cellulose and nutrient salts, at least one sterilising vessel (10) equipped with agitation means and preceded by a mixer (7) including a supply duct (8) for sterilising agent, a pre-fermentation arrangement, advantageously comprising a plurality of pre-fermenters (11, 12, 13), each having a capacity that is about 10 times larger than that of the preceding fermenter, and a main fermentation arrangement comprising a plurality of fermenters (14), the pre- and main fermenters including supply means for oxygen-containing gases, agitating and mixing organs, aeration means, means for controlling the temperature and the pH and, if desired, foam breakers, as well as, furthermore, means for separating the solids from the cellulase-containing solution, advantageously comprising a filtering basin (16) and a centrifuge (17), and, if desired, an ultrafiltration plant (29) for concentrating the cellulase-containing solution as well as a vessel (32) for precipitating the cellulase.

0 062 026